# EUROPEAN PATENT APPLICATION

(11) **EP 3 563 875 A1**
(43) Date of publication of application: **06.11.2019**
(21) Application number: 18170735.7
(22) Date of filing: 04.05.2018
(51) Int. Cl.: A61K 51/02, A61K 51/12

(54) **RADIUM FOR RADIONUCLIDE THERAPY, IN COMBINATION WITH CALCIUM METABOLISM AFFECTING TREATMENT**

(71) Applicant: Ceské vysoké ucení technické v Praze, 160 00 Praha 6 - Dejvice (CZ); UJV Rez, A.S., 25068 Husinec-Rez (CZ)
(72) Inventor: Kozempel, Ján, 688 01 Uherský Brod (CZ); Vlk, Martin, 908 46 Unín (SK); Mi olová, Petra, 542 33 Rtyn v Podkrkono í (CZ); Kukleva, Ekaterina, 360 01 Karlovy Vary (CZ); Jandová, Ludmila, 130 00 Praha 3 - Vinohrady (CZ); Merhautová, Hana, 250 92 estajovice (CZ); Ficenzová, Karolína, 110 09 Praha 1 - Staré m sto (CZ)
(74) Representative: Hartvichova, Katerina

(57) **Abstract**

The present invention relates to a set for radionuclide therapy, comprising a component A and a component B, for use in the treatment of bone diseases by administration of the component B in a dosage ensuring a higher than physiological concentration of a substance affecting Ca²⁺ metabolism in the organism and/or in the compartment treated, and co-administration of the component A, wherein the component A comprises a water-soluble radium salt containing at least one radionuclide of radium, and the component B comprises at least one substance affecting Ca²⁺ metabolism in the organism, selected from the group comprising water-soluble salts of alkaline earth metals, calciferols, calcitonins, thyroid and parathyroid gland hormones, amino acids and salts thereof, peptides having a chain length of 2 to 100 amino acids and salts thereof, bisphosphonates of the general formula (I), 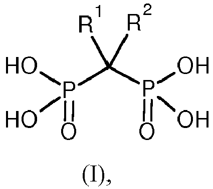 wherein R¹ is -H, -OH, -Cl;
R² is -CH₃, -Cl, -CH₂CH₂NH₂, -(CH₂)₅-NH₂, -(CH₂)₃-NH₂, 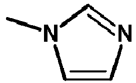 CH₂CH₂N(CH₃)-(CH₂)₄CH₃;
and combinations thereof.

## Description

### Field of Art

The present invention relates to the use of a set for radionuclide therapy in nuclear medicine in the treatment of oncological diseases, especially bone tumours and metastases.

### Background Art

Radionuclide therapy is an important tool in the treatment of oncological diseases, autoimmune diseases (especially of joints), or palliative therapy. Simple formulations of such drugs are known, such as ionic or colloidal solution formulations ^{99m}Tc, ¹⁵³Sm, ¹⁶⁹Er, ¹⁷⁷Lu, ³²P, ²²³Ra, and more with excipients such as water for injections, sodium chloride, buffers (citrate, phosphate), dilute acids and the like. These excipients ensure their biocompatibility and allow intravenous or other administration of a radiopharmaceutical. Studies on the compatibility of such pharmaceuticals are not usually available and therefore these medicines should not be mixed with other medicinal products (active substances). It is well known that the interspecies variability and variability among individuals of the same species is considerable. This makes it virtually impossible to compare individual clinical and preclinical studies.

Therapeutically usable radioisotopes are ²²³Ra (t_{1/2} = 11.4 day), ²²⁴Ra (t_{1/2} = 3.66 day), ²²⁵Ra (t_{1/2} = 14.9 day), which are administered intravenously. In a human study after intravenous injection, radium-223 is eliminated from the blood and is deposited primarily in the bones and bone metastases or is excreted by the intestine. Based on available data on a product containing radium-223 dichloride, fifteen minutes after the injection, about 20 percent of the activity remains in the blood. After four hours, approximately four percent of the administered activity remains in the blood, and the activity decreases to less than one percent 24 hours after injection. The volume of distribution was higher than the blood volume, indicating distribution into peripheral compartments. Approximately ten minutes after injection, an activity was observed in the bones and the intestine. Four hours after injection, the level of activity in the bones was 44 % to 77 %. No significant uptake was observed within four hours after injection in other organs such as the heart, liver, kidney, urinary bladder, and spleen. The whole-body measurement seven days after injection (after adjustment due to breakdown) shows that on average 76 % of the activity administered was excreted from the body. The rate of elimination of radium-223 dichloride from the gastrointestinal tract is affected by the high variability of intestinal transit across population with a normal range of emptying once a day to once a week.

From the point of view of the use of a non-isotopic carrier for radium (e.g. calcium) it may be expected, according to the present knowledge, that radium will take over the chemical properties of the carrier and, in the case of simultaneous administration, there will be a competition between the carrier and radium and thus radium is displaced (Majer V. a kol., Základy jaderné chemie, 2. přepracované vydání, SNT, Praha 1981).
According to the state of the art, the effect of administering substances affecting calcium metabolism on the metabolism of radium is unknown. Because interaction with calcium and phosphates cannot be ruled out, it is recommended to stop taking preparations with these substances and/or vitamin D several days before starting the treatment with radium. Given the chemical analogy between calcium and radium, radium may be expected to be subject to similar metabolic pathways in the body and will be eliminated more quickly from organisms due to the competition with calcium.

Parathormone (PTH), vitamin D, calcitonin (peptide with a chain length of 32 amino acids) and their target organs (skeleton, kidney, and intestine) are responsible for maintaining the calcium homeostasis in the human body, the metabolism of PTH and vitamin D being interconnected, especially with PTH increasing the production of active vitamin D, while vitamin D deficiency reduces the effect of PTH at the same time.
Vitamin D, after activation to calcitriol (1α, 25-dihydroxycholecalciferol), substantially affects the calcium and phosphorus homeostasis. Physiological concentrations of calcitriol can accelerate the tumour cell growth, due to the binding of calcitriol to a specific receptor (e.g. mamma carcinoma, melanomas) and pharmacological doses (2 - 3 mg) can inhibit growth [Klener P.: Protinádorová terapie. Galén, Praha 1996]. Calcitriol increases the resorption of Ca²⁺ and phosphates from the intestine and their reabsorption in the kidneys, as a result of this increased concentration of Ca²⁺ ions and phosphates in the blood, the formation of hydroxyapatites in the bone tissue elevates [Lüllmann H. et al. Barevný atlas farmakologie. Grada, Praha 2012]. Also other thyroid and parathyroid gland hormones are known to have an effect, for example, on accelerating the reabsorption of bone tissue. PTH (peptide with a chain length of 84 amino acids) actively increases Ca²⁺ level in blood.

Bisphosphonates inhibit *in vitro* precipitation of calcium phosphate and its conversion into hydroxyapatite, by attenuation of proteosynthesis and depression of lysosomal enzyme release, formation of prostaglandins and activity of pyrophosphatase and glycolysis. Bisphosphonates do not affect the endocrine functions of the organs, but act primarily in the skeletal area - in the place of increased resorption. Mechanisms of action of bisphosphonates can be divided according to where the action takes place:
a) at a tissue level where mineralization, bone mass, and ratio of mineral content to bone density are increased;
b) at a cellular level where localization of bisphosphonates on the surface of osteoclasts and inhibition of ruffled membranes occur;
c) at a molecular level where they inhibit farnesyl diphosphate synthases, reduction of geranylgeranyl diphosphate and pranylation of small GPTases, disrupt the adhesion and migration of osteoclasts, which can result in apoptosis.

Bisphosphonates are classified as first generation phosphonates - without amino groups (e.g. tiludronic acid, clodronic acid, and etidronic acid); second generation phosphonates - containing amino groups (e.g. pamidronic acid, alendronic acid, and ibandronic acid), and third generation phosphonates - containing nitrogen heterocycle (e.g. risedronic acid and zoledronic acid) in their structure. The side chains of bisphosphonates may enter metabolism, especially with the first generation, where osteoclast degradation leading to ATP analogues has been observed. This biodegradation does not occur with aminobisphosphonates.
For oral administration, bioavailability ranges from 1 to 10 %, wherein the absorption takes place in the gastrointestinal tract (stomach, small intestine). Bioavailability is negatively affected by the current food intake, especially food rich in calcium and iron ions. The biological elimination half-life of bisphosphonates ranges from 0.5 to 2 hours, wherein plasma clearance is between 6 and 10 hours. Up to 60 % of resorbed bisphosphonates are implemented into bone tissue, with the rest eliminated. The time for which bisphosphonates are incorporated into bone tissue is over 10 years (up to lifetime). Application of bisphosphonates and their intervention in bone resorption and formation is associated with overall changes in calcium metabolism. Preferred is intravenous administration with a dosage recommendation according to Table 1; the oral form is not available for all bisphosphonates [Aapro M. et al.: Annals of Oncology 19: 420-432, 2008]. In almost all cases, a renal function check is performed before starting the therapy and before each application. Despite very good tolerance of the body to therapy, adverse effects may occur, such as renal toxicity, gastrointestinal tract problems, and osteonecrosis of the jaw [Sláma O.: Paliatívna medicína a liečba bolesti 2(2) 2009].

**Table 1. Dosage of bisphosphonates**

| Bisphosphonate | Intravenous infusion administration | Peroral administration |
|---|---|---|
| clodronic acid | 900 mg/4h every 3-4 weeks | 1024 - 2400 mg/day |
| pamidronic acid | 90 mg/2h every 3-4 weeks | n.a. |
| zoledronic acid | 4 mg/15 min every 3-4 weeks | n.a. |
| ibandronic acid | 6 mg/15 min every 3-4 weeks | 50 mg/day |
| etidronic acid | 200 mg/ 2 times a day | - |

Furthermore, it is known that certain amino acids or their salts may form complexes with Ca2⁺ or affect its metabolism - e.g. L-lysine (Civitelli, R; Villareal, D.T; Agnusdei, D; Nardi, P; Avioli, L. V; Gennari, C.: Dietary L-lysine and calcium metabolism in humans. Nutrition. 8(6):400-405, (1992)), arginine (Antonilli, M; Bottari, E; Gentile, M. R. F. & L.: Complex formation between arginine and calcium (II) and magnesium (II), Chemical Speciation & Bioavailability, 21:1, 33-40, (2009), DOI: 10.3184/095422909X418502; Maeda, M; Okada, K; Tsukamoto, Y; Wakabayashi , K; Ito, K.: Complex formation of calcium(II) with amino acids under physiological conditions. J. Chem. Soc., Dalton Trans. 2337-2339, (1990)).

In clinical practice, administration of abiraterone acetate (1000 mg/day) with prednisone (5 mg/day) has recently been reported in asymptomatic patients under androgen deprivation therapy (ADT), in symptomatic patient, the application of docetaxel with prednisone is recommended (Matějů M.: Vhodný pacient pro léčbu abirateron acetátem, Klin. Onkol. 2017; 30(1):59-62). The European Medicines Agency (EMA) has recommended not using ²²³Ra-chloride in combination with prednisone, prednisolone and abiraterone acetate, because of the increased risk of fractures and deaths. The ongoing clinical evaluation of patients with metastatic prostate cancer concluded that 34.7 patients treated with a combination of ²²³RaCl₂ (Xofigo), abiraterone acetate, prednisone and prednisolone died. Patients who received placebo with prednisone and prednisolone and abiraterone acetate died 28.2 percent (Informační dopis - Xofigo, Státní ústav pro kontrolu léčiv. Státní ústav pro kontrolu léčiv [online]. Copyright© 2001 [cit. 12.04.2018]. Available at http://www.sukl.cz/leciva/informacni-dopis-xofigo-3).

In the state of the art there is still no therapeutic use of radioactive Ra in which the biodistribution of Ra in the organism would be modulated so as to increase uptake of Ra in the bones, and at the same time Ra would be rapidly eliminated from other organs where its presence is undesirable.

### Description of the Invention

The present invention relates to a therapeutic use of a set for radionuclide therapy, which modulates biodistribution of Ra in the organism. The use of the set of the present invention surprisingly increases the uptake time of Ra radionuclides in the organism, which allows a higher uptake of Ra in the bones, while reducing the Ra uptake time in other organs (e.g. spleen or liver), thus the action time of Ra radionuclides in the target bone tissue increases, with simultaneous rapid elimination of this substance from other organs where its presence is undesirable. The essence of the present invention lies in a paradoxical and utterly unexpected finding that after the addition of supraphysiological concentration of substances affecting Ca²⁺ metabolism, while the organism as such already contains Ca²⁺ ions (at least in trace amounts), paradoxically, there is an increased Ra uptake in bone tissue, although it should lead to its increased elimination, while at the same time Ra occurrence in other organs decreases and thus it is eliminated faster. Due to this unexpected effect, neither a stabilizer nor Ra radionuclide carrier, which has been used in the prior art to stabilize Ra in solutions, is needed.
The principle is to increase the concentration of the substance(s) affecting the calcium metabolism in the organism above its/their physiological concentration up to the maximum tolerated concentration (e.g. the maximum recommended dose of zoledronic acid for human administration is 4 mg per intravenous infusion (NOAEL 0.02 mg/kg/day, intravenously or subcutaneously), but up to 16 mg (Novartis, zoledronic acid SPC) was administered in clinical studies). The presence of substances affecting the calcium metabolism in the body at a higher than physiological concentration modulates the Ra uptake mechanisms in the organism.
The administration of Ra to the body then leads to its increased sorption to bone tissues and its rapid elimination from other tissues of the organs. The combination of increased concentration of substances affecting Ca²⁺ metabolism in the body and Ra radionuclide leads to an unexpected synergistic effect of increasing the Ra uptake time in the bones and its faster elimination from other organs. Those skilled in the art would not expect this effect; on the contrary, the state of the art would lead them to the assumption that Ra²⁺ assumes the chemical properties of Ca²⁺, and in the case of concurrent administration, a competition between Ca²⁺ and Ra²⁺ occurs, thereby displacing Ra²⁺. Also, in the case of physiological or pathophysiological concentration of Ca²⁺ in the organism, one skilled in the art would expect that Ca²⁺ and Ra²⁺ competition will occur when other Ca2⁺ modulating substances are administered, and thus increased displacement of Ra²⁺ from the organism, or preferentially incorporation of Ca²⁺ in the bones.

The object of the present invention is a set for radionuclide therapy for use in the treatment of bone diseases, particularly oncological diseases of bone, the set comprising a component A, which comprises a water-soluble radium salt containing at least one radionuclide of radium, and a component B, which comprises at least one substance affecting Ca²⁺ metabolism in the organism, selected from the group comprising water-soluble salts of alkaline earth metals, calciferols, calcitonins, thyroid and parathyroid gland hormones, amino acids and salts thereof, peptides having a chain length of 2 to 100 amino acids and salts thereof, bisphosphonates of the general formula (I), 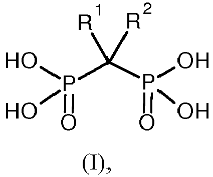 wherein R¹ is -H, -OH, -Cl;
R² is -CH₃, -Cl, -CH₂CH₂NH₂, -(CH₂)₅-NH₂, -(CH₂)₃-NH₂, CH₂CH₂N(CH₃)-(CH₂)₄CH₃; 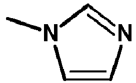 and combinations thereof,
wherein the component A is preferably in the form of a solution, preferably the solvent is water or isotonic sodium chloride solution, and its specific volume activity is preferably in the range of 0.5 MBq/mL to 10 MBq/mL, preferably the solution of component A has radium concentration in the range of 0.3 nM to 30 nM,
and wherein the component A and component B are administered so that, during the presence of the component A in the organism, the concentration of the substance affecting Ca²⁺ metabolism in the organism is higher than its physiological concentration. In one preferred embodiment, this can be achieved by administering the component B first (single or multiple doses) and only subsequently, upon reaching the supraphysiological (higher than physiological) concentration of the substance affecting Ca²⁺ metabolism in the organism, by administration of a single dose of the component A.
In another embodiment, the component A and the component B may be co-administered simultaneously, in which case it is essential that the concentration of the substance affecting Ca²⁺ metabolism in the organism in the presence of the component A in the organism reached the supraphysiological concentration in the treated compartment. The possibility is, for example, to inject component B directly into the treated part (compartment) of the body.
In another embodiment, the component A may be administered first and, subsequently, the component B. The condition is again that, in the presence of component A in the organism, the concentration of the substance affecting Ca²⁺ metabolism in the organism reached the supraphysiological concentration in the treated compartment. The possibility is, for example, to inject component B directly into the treated part (compartment) of the body.
The time delay between administration of the component A and the component B depends on the dose of the substance affecting Ca²⁺ metabolism in the organism in component B, the route of administration and time over which the substance reaches higher than the physiological concentration in the organism and/or the compartment being treated, and their pharmacokinetic properties. Usually, the delay in administration is at least 1 hour, several days, but exceptionally more than 14 days.
Higher than physiological concentration of the substance affecting the metabolism of calcium ions in the body is individual for each substance, and the person skilled in the art is able to determine which concentration of the respective substance may be considered as supraphysiological. Physiological concentrations of substances affecting metabolism of calcium ions in the body are shown in Table 2 below.

**Table 2 of physiological concentrations of substances affecting the metabolism of calcium(II) ions in the body:**

| Substance | Expected physiological concentration in human body (plasma) | Reference |
|---|---|---|
| Ca²⁺ | 2,15-2,61 mmol/L | TROJAN, S. et al. Lékařská fyziologie. 4. vydání. Praha: Grada, 2003. ISBN 80-247-0512-5. |
| Mg²⁺ | 0,66-0,94 mmol/L | TROJAN, S. et al. Lékařská fyziologie. 4. vydání. Praha: Grada, 2003. ISBN 80-247-0512-5. |
| Sr²⁺ | 0,21 - 1,08 pmol/L | Somarouthu, S., Ohh, J., Shaked, J., Cunico, R.L., Yakatan, G., Corritori, S., Tami, J. & Foehr, E.D.: Quantitative bioanalysis of strontium in human serum by inductively coupled plasma-mass spektrometry. Future Sci. OA (2015) 1(4), FSO76, DOI: 10.4155/fso.15.76. |
| vitamine D | 50 - 125 nmol/L | ROCHE Diagnostics, COBAS® Vitamin D total, method sheet. Ref.: 05894913 190, Rev. 2018-01, V 7.0 - in Czech. |
| calcitonine | muži: 2,43 - 4,18 pmol/L | ROCHE DIAGNOSTICS COBAS® Calcitonin, method sheet, Ref. 06445853 190, Rev. 2016-03, V 4.0 in Czech |
| | ženy: 1,51 - 2,87 pmol/L | |
| parathormone | 1,6-6,9 pmol/L | ROCHE DIAGNOSTICS COBAS® PTH, method sheet, Ref. 11972103 122, Rev. 2017-02, V 24.0 in Czech |
| zoledronic acid | 0 | - |

Preferably, the upper limit of the concentration of the substance affecting Ca²⁺ metabolism in the body and/or in the compartment being treated is the maximum tolerated concentration of the substance in the body. This maximum tolerated concentration is known to the person skilled in the art. More preferably, the upper limit of the concentration of the substance affecting Ca²⁺ metabolism is below its toxic concentration, more preferably below the maximum tolerated concentration, but always higher than its physiological concentration.

The component A may further comprise other excipients selected from the group comprising water for injections, saccharides of a pentose or hexose group, and pharmaceutically acceptable buffers. Person skilled in the art would be able to choose a suitable carbohydrate and/or buffer as an excipient for component A for intravenous application, without exercising an inventive activity. The component B comprises at least one substance affecting the metabolism of Ca²⁺, preferably in a weight range of 0.001 mg to 1000 mg per dose. The optimal amount of substance affecting Ca²⁺ metabolism in the component B depends on the particular type of the substance and on the target organism. A person skilled in the art would be able to choose an appropriate amount of the substance affecting Ca²⁺ metabolism without exercising an inventive activity, since the maximum tolerated administered concentrations or total doses of individual substances affecting Ca²⁺ metabolism are generally known (e.g. Dietary Reference Intakes for Calcium and Vitamin D. Institute of Medicine (US) Committee to Review Dietary Reference Intakes for Vitamin D and Calcium; Ross, A.C., Taylor, C.L., Yaktine, A.L., et al., Editors. Washington (DC): National Academies Press (US); 2011). It should be clear that in the case of use according to the present invention, the administration of the component B is temporary and is favourably limited by the physical half-life of radium isotopes.

Radium salts are in particular salts of Ra²⁺ and of inorganic acids or organic monocarboxylic, dicarboxylic and tricarboxylic (C1 to C10) acids, preferably Ra salts are carbonate, halides, nitrate, phosphates, acetate, formate, citrate, oxalate, lactate, and salts of amino acids or peptides having a chain length of 1 to 50 amino acids, more preferably Ra salts are selected from the group consisting of RaCO₃, Ra(NO₃)₂, RaCl₂, RaBr₂, RaI₂, RaF₂, Ra(CH₃COO)₂, most preferably the Ra salt is RaCl₂.

A water-soluble Ra salt is a salt whose solubility is greater than 5 mg/L, preferably > 20 mg/L (RaCO₃), more preferably > 100 g/L (e.g. Ra (NO₃)₂), but most preferably > 150 g/L (e.g. RaCl₂) at 20 °C.
Salts of alkaline earth metals are in particular their salts with inorganic acids or organic monocarboxylic, dicarboxylic and tricarboxylic (C1 to C10) acids, preferably alkaline earth metal salts are carbonates, halides, nitrates, phosphates, acetates, formates, citrates, oxalates, lactates, and salts of amino acids or peptides having a chain length of 1 to 50 amino acids, more preferably the alkaline earth metals forming salts are selected from the group consisting of magnesium, calcium, and strontium. Most preferably, the alkaline earth metal salt is CaCl₂.
A water-soluble alkaline earth metal salt is a salt whose solubility is greater than 400 g/L at 20 °C. The component B may be pure, in the form of a solution or in the form of a mixture with pharmaceutically acceptable excipients, depending on the form of administration (dosage form).

The component B may also be administered, for example, as part of a food or beverage. Preferably, the solvent for component B is water, triglyceride oil, isotonic NaCl solution or polyethylene glycol with a maximum molecular weight (MW) of 4,000 g/mol. A solution of the component B is, for example, an aqueous solution of alkaline earth metal salts or an oily solution of calciferols, calcitonins and thyroid hormones.
The component B may further comprise an effective amount of inhibitors of osteoclast activity for the treatment of oncological diseases, preferably phosphonates selected from the group comprising clodronate, pamidronate, ibadronate, aledronate, risedronate, oxidronate, medronate, etidronate, tiludronate, and zoledronate, more preferably phosphonates are in a hydrated form or in the form of sodium salt.
Excipients include preservatives, stabilizers, humectants and/or emulsifiers, solubilizers, salts to regulate osmotic pressure and/or buffers, in particular, for example, saccharides of a pentose or hexose group, buffers (e.g. phosphate, citrate, HEPES), amino acids, pharmaceutically acceptable carriers (e.g. microcrystalline cellulose, colloidal SiO₂), wherein the route of administration of component B may be intravenous, subcutaneous, nasal or oral in the form of sodium salts (e.g. L-thyroxine sodium salt pentahydrate).

The component A may be administered prior to administration of component B, administered together with the component B and/or after administration of the component B. Preferably, the component A is administered after administration of the component B. The time interval between administration of the component A and administration of the component B depends on the type of substance that affects calcium metabolism in the body and on its biological half-life. The component B may be administered prior to, at the same time and after administration of the component A for a more preferred effect on metabolism of the component A.
Achieving a higher than physiological concentration of the substance affecting calcium metabolism in the body triggers metabolic pathways modulating the absorption and biodistribution of the component A. It is also preferred to maintain a higher than physiological concentration of the substance affecting calcium metabolism in the body, over the time of retainement of the component A in the organism, more preferably until ten biological half-lives of the component A.
In addition to modulating effect of the component B on biodistribution and metabolism of the component A, there is a synergistic combined radio-chemotherapeutic effect of the component A and the component B, when the component B reaches its therapeutic concentration. The component A and/or the component B may be administered in one single dose or repeatedly in regularly repeated doses to create and maintain selected concentrations of the relevant component in the body. In one preferred embodiment, the component B is administered repeatedly to create and maintain selected concentrations of a substance affecting calcium metabolism in the organism, and the component A is administered once or repeatedly, most preferably the component B is administered repeatedly to create and maintain selected concentration of a substance affecting calcium metabolism in the organism and the component A is administered in one single dose.

While the component A contains its own therapeutic radionuclides, the component B contains substance/substances capable of affecting/ modulating metabolism of Ca²⁺ ions in the organism and their unexpected synergistic effect results in influencing the biodistribution of Ra²⁺ in the organism.

In one embodiment, the component A is administered first, followed by administration of the component B until a higher than physiological concentration of the substance affecting the metabolism of calcium in the organism is reached. Preferably, administration of the component A is in a single dose. Component B may be administered in single dose or in multiple doses. Maintaining higher than physiological concentration of the component B in the organism is preferred throughout the whole presence of Ra radionuclide in the organism.
The time delay between administration of the component A and of the component B in this embodiment is preferably within 24 hours, more preferably within 12 hours, preferably until the biological half-life of the component A is reached. However, the component B may be effectively administered also later, at most until ten biological half-lives of the component A are reached.

In another embodiment, component B is administered first until a higher than physiological concentration of the substance affecting the metabolism of calcium in the organism is reached, followed by administration of the component A. Preferably, administration of component A is in a single dose. Component B may be administered in a single dose or in multiple doses.
In order to maintain a higher than physiological concentration of component B in the organism throughout the presence of Ra radionuclide in the organism, it is preferable, after administration of component A, to administer component B again (even repeatedly) until ten biological half-lives of component A are reached.

In another embodiment, components A and B are co-administered simultaneously, wherein component B is administered in an amount that provides for a higher than physiological concentration of the substance affecting the metabolism of calcium in the organism and/or in the compartment being treated. It is preferable to maintain the higher than physiological concentration of this substance in the organism throughout the presence of Ra radionuclide in the organism, that is after simultaneous co-administration of components A and B to administer the component B again (even repeatedly) until ten biological half-lives of component A are reached.

In one embodiment, Ra radionuclide is selected from the group comprising ²²³Ra, ²²⁴Ra, ²²⁵Ra isotopes.
In one preferred embodiment, the substance influencing calcium metabolism in the body is selected from water-soluble Mg²⁺, Ca²⁺ Sr²⁺ salts, vitamin D, calcitonin, parathormone, thyroxine, triiodothyronine, amino acids, especially L-lysine and/or arginine, or peptides having a chain length of 2 to 50 amino acids, and zoledronic acid, and combinations thereof, more preferably the substance influencing calcium metabolism in the body of component B is selected from MgCl₂, CaCl₂, SrCl₂, vitamin D, calcitonin, parathormone or combinations thereof, most preferably the substance affecting calcium metabolism in the body of component B is CaCl₂.

In one embodiment, the component A and/or the component B further comprises up to 99.9 wt. %, preferably 5 to 90 wt. %, more preferably 20 to 70 wt. % of a pharmaceutically acceptable excipient.

Pharmaceutically acceptable excipients are, for example, preservatives, stabilizers, humectants and/or emulsifiers, solubilizers, salts to regulate osmotic pressure, and/or buffers. Preferably, the pharmaceutically acceptable excipient is selected from the group comprising NaCl, citric acid and/or salts thereof, acetic acid and/or salts thereof, phosphoric acid and/or salts thereof, microcrystalline cellulose, saccharides of a pentose and hexose group, colloidal SiO₂, and pH-adjusting agents (NaOH, KOH).

In a preferred embodiment, the set for radionuclide therapy is particularly intended for use in the treatment of bone cancer diseases, particularly in the treatment of bone metastases, e.g. for the treatment of adult men suffering with castration-resistant prostate cancer, symptomatic bone metastases and without known visceral metastases.

In one embodiment, component A is administered intravenously and component B is administered orally and/or subcutaneously and/or nasally and/or intravenously, wherein the component B may be in various dosage forms - e.g. solution, pill, powder or aerosol.

In one embodiment, the set for radionuclide therapy of the present invention for use in treatment in a dosage regimen comprising administering the component B at least twice a week at least until a higher than physiological concentration of the substance affecting calcium metabolism in the organism is reached, and in subsequent single-dose administration of the component A at a higher than physiological concentration of the substance affecting calcium metabolism in the organism, and, eventually, subsequent administration of the component B, preferably at least twice a week, to maintain its higher than physiological concentration throughout the time of presence of Ra radioisotope in the organism, preferably during ten biological half-lives of the component A.
The administration of the component B preferably takes place in the time interval of from 0.5 hours to 11 days after administration of the component A, at the latest within ten biological half-lives of the component A. The physiological concentration of a substance affecting calcium metabolism in the organism of component B for particular substances is known to the skilled in the art.
The maximum concentration of the substance affecting calcium metabolism in the body of component B in the organism usually does not exceed the maximum recommended dosage but depends on the sex, age and condition of the patient (e.g. typically 4 mg for zoledronic acid intravenously in a single dose, 3 to 100 µg orally/day for vitamin D, up to 1,000 mg intravenously for calcium chloride). The maximum tolerated concentrations of substances influencing calcium metabolism in the body are known to the skilled in the art.

### Brief Description of Figures

Fig. 1: 24 h. biodistribution of ²²³Ra with CaCl₂
Fig. 2: 96 h. biodistribution of ²²³Ra with CaCl₂
Fig. 3: 24 h. biodistribution of ²²³Ra with vitamine D3
Fig. 4: 96 h. biodistribution of ²²³Ra with vitamine D3
Fig. 5: 24 h. biodistribution of ²²³Ra with zoledronic acid
Fig. 6: 96 h. biodistribution of ²²³Ra with zoledronic acid
Fig. 7: 24 h. biodistribution of ²²³Ra without in vivo modulating substances
Fig. 8: 96 h. biodistribution of ²²³Ra without in vivo modulating substances

### Examples

### General description of experiments

The study was performed in female outbred CD1 mice imported from Charles River breeds. Animals were housed in groups of five and acclimated over five days. After acclimatization, the animals were divided into four groups. The day before the experiment the feed was taken away and at the beginning of the experiment itself the animals were weighed and placed in cages individually on a nonwoven fabric.
After administration of ²²³Ra (ca 200 kBq/animal in 0.05 ml of physiological saline) through *vena caudalis,* excrements were collected after one and four hours; after 24 hours, five mice were sacrificed from each group, another five were sacrificed after 96 hours. Blood, heart, lung, liver, pancreas, spleen, kidney, urinary bladder, wall of the colon, colon content, stomach, stomach content, thyroid gland, brain, femur, ovaries, parts of skin and muscles, and excrements from each mouse were removed. The organs and excrements in the measuring vessels were weighed and measured in a well of NaI (T1) scintillation detector.

### Example 1: Study of biodistribution and elimination of ²²³Ra when using ²²³RaCl₂ as component A and CaCl₂ as component B of the set for radionuclide therapy

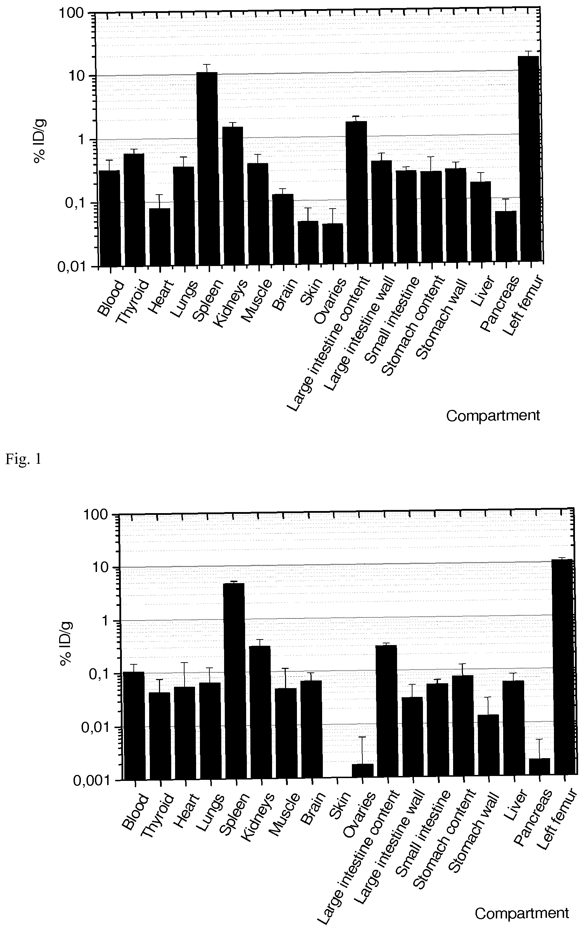

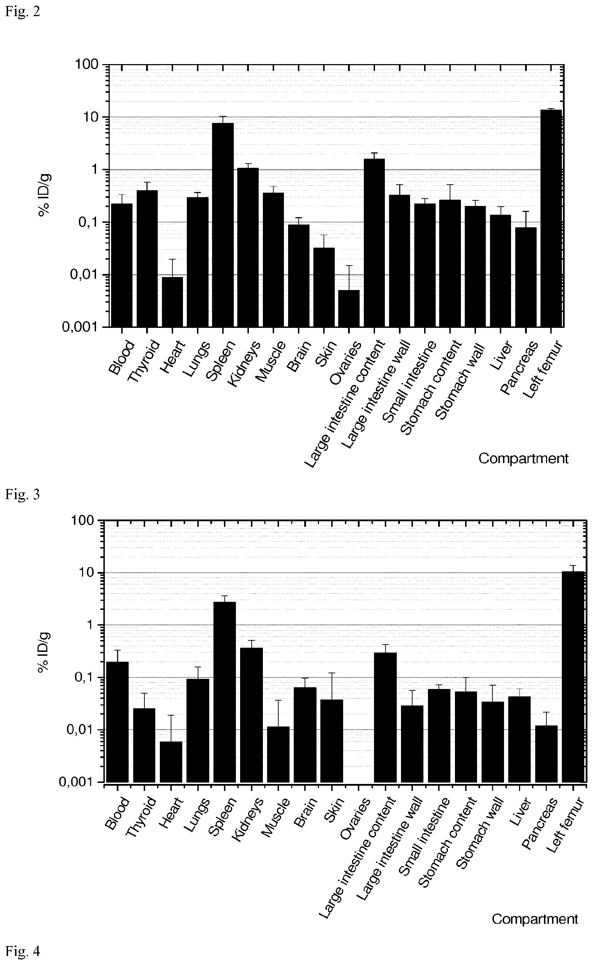

Ten mice were medicated with CaCl₂ in the concentration of 1.11 g/L for one week prior to administration of ²²³Ra. The solution was used to feed *per os ad libitum* throughout the experiment. Biodistribution of ²²³Ra after 24 and 96 hours following administration is shown in Table 3 and Figures 1 and 2.

**Table 3. Biodistribution of ²²³Ra with CaCl₂**

| Organ | %ID/g after 24 h | SD | %ID/g after 96 h | SD |
|---|---|---|---|---|
| Thyroid | 0.58 | 0.12 | 0.04 | 0.03 |
| Spleen | 10.,71 | 3.69 | 4.62 | 0.50 |
| Kidneys | 1.47 | 0.27 | 0.30 | 0.11 |
| Large intestine content | 1.69 | 0.36 | 0.29 | 0.03 |
| Left femur | 16.51 | 3.62 | 10.80 | 1.15 |
| Liver | 0.18 | 0.08 | 0.06 | 0.02 |

In total after 24 h eliminated in excrements 31.93 ± 8.57 % ID/g ²²³Ra, in urine 16.53 ± 3.99 % ID ²²³Ra.
In total after 96 h eliminated in excrements 24.90 ± 2.14 % ID/g ²²³Ra, in urine 18.43 ± 4.32 % ID ²²³Ra.

### Example 2: Study of biodistribution and elimination of ²²³Ra when using ²²³RaCl₂ as component A and vitamin D3 as component B of the set for radionuclide therapy

15 mice were pre-medicated with vitamin D3, 160 IU/mouse (0.02 mL of triglyceride oil solution of vitamin D3; 0.2 mg/ml) administered *per os* with part of the feed, three times a week for one week prior to administration of ²²³Ra. Five of these mice were eliminated during the experiment because they refused vitamin intake. Biodistribution of ²²³Ra after 24 and 96 hours following administration is shown in Table 4 and Figures 3 and 4.

**Table 4. Biodistribution of ²²³Ra with vitamin D3**

| Organ | %ID/g after 24 h | SD | %ID/g after 96 h | SD |
|---|---|---|---|---|
| Thyroid | 0.40 | 0.18 | 0.03 | 0.02 |
| Spleen | 7.55 | 2.76 | 2.70 | 0.91 |
| Kidney | 1.06 | 0.23 | 0.36 | 0.15 |
| Large intestine content | 1.57 | 0.52 | 0.29 | 0.13 |
| Left femur | 13.43 | 1.10 | 10.37 | 3.52 |
| Liver | 0.13 | 0.06 | 0.04 | 0.02 |

In total after 24 h eliminated in excrements 17.28 ± 2.00 % ID/g ²²³Ra, in urine 15.92 ± 3.05 % ID ²²³Ra.
In total after 96 h eliminated in excrements 23.56 ± 10.95 % ID/g ²²³Ra, in urine 18.60 ± 4.54 % ID ²²³Ra.

### Example 3: Study of biodistribution and elimination of ²²³Ra when using ²²³RaCl₂ as component A and zoledronic acid of formula (II) as component B of the set for radionuclide therapy

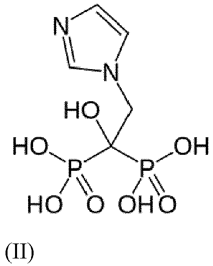

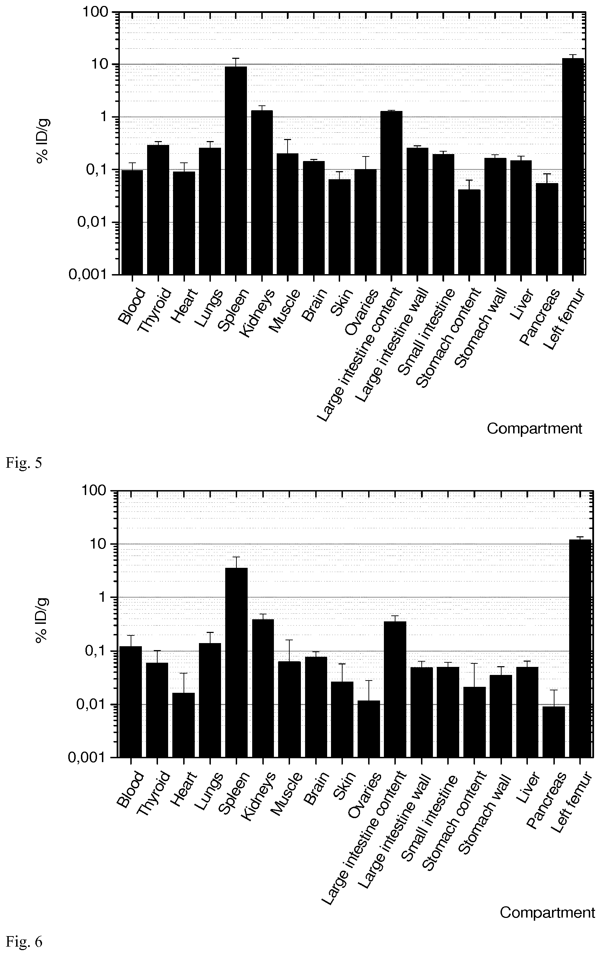

Ten mice were medicated with zoledronic acid; 0.1 mL preparation/mouse was taken from the 4 mg/100 mL infusion solution and administered subcutaneously twice a week for one week before administration of ²²³Ra. Biodistribution of ²²³Ra after 24 and 96 hours following administration is shown in Table 5 and Figures 5 and 6.

**Table 5. Biodistribution of ²²³Ra with zoledronic acid**

| Organ | %ID/g after 24 h | SD | %ID/g after 96 h | SD |
|---|---|---|---|---|
| Thyroid | 0.29 | 0.06 | 0.06 | 0.04 |
| Spleen | 8.88 | 4.65 | 3.51 | 2.22 |
| Kidney | 1.30 | 0.38 | 0.38 | 0.11 |
| Large intestine content | 1.27 | 0.08 | 0.35 | 0.11 |
| Left femur | 12.78 | 2.77 | 11.88 | 1.87 |
| Liver | 0.15 | 0.03 | 0.05 | 0.02 |

In total after 24 h eliminated in excrements 19.27 ± 3.25 % ID/g ²²³Ra, in urine 17.50 ± 3.21 % ID ²²³Ra
In total after 96 h eliminated in excrements 27.65 ± 12.62 % ID/g ²²³Ra, in urine 22.66 ± 9.62 % ID ²²³Ra.

### Example 4: Study of biodistribution and elimination of ²²³Ra when using only ²²³RaCl₂ as component A without using component B of the set (control group)

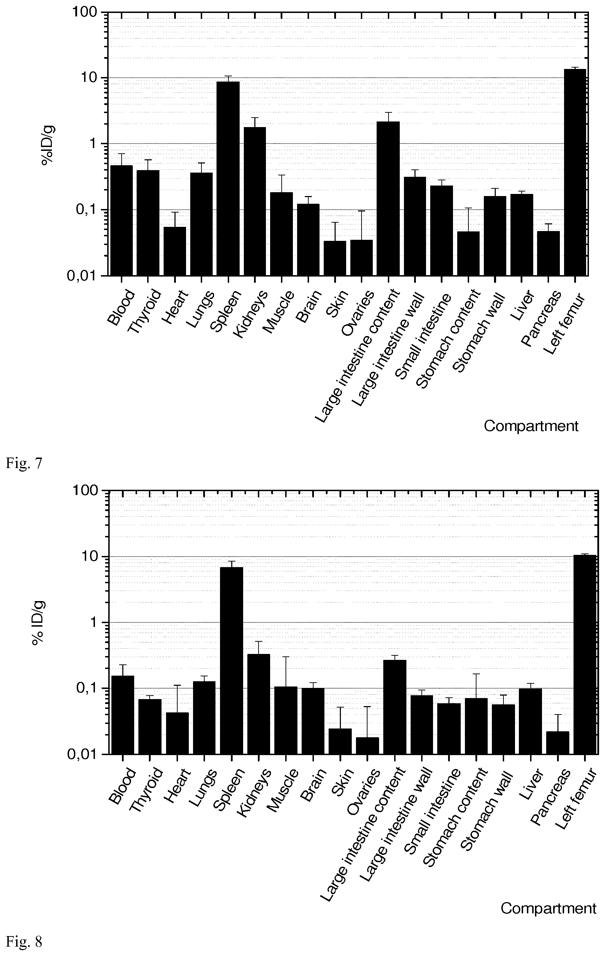

Ten mice were used as a control group. Biodistribution of ²²³Ra after 24 and 96 hours following administration is shown in Table 6 and Figures 7 and 8.

**Table 6. Biodistribution of ²²³Ra**

| Organ | %ID/g after 24 h | SD | %ID/g after 96 h | SD |
|---|---|---|---|---|
| Thyroid | 0.39 | 0.18 | 0.07 | 0.01 |
| Spleen | 8.59 | 2.09 | 6.77 | 1.68 |
| Kidney | 1.76 | 0.74 | 0.32 | 0.19 |
| Large intestine content | 2.14 | 0.86 | 0.27 | 0.05 |
| Left femur | 13.37 | 1.14 | 10.33 | 0.65 |
| Liver | 0.17 | 0.02 | 0.10 | 0.02 |

In total after 24 h eliminated in excrements 33.54 ± 16.22 %ID/g ²²³Ra in urine 16.24 ± 2.29 %ID ²²³Ra.
In total after 96 h eliminated in excrements 36.43 ± 12.31 %ID/g ²²³Ra in urine 17.73 ± 2.34 %ID ²²³Ra.

### Example 5: Study of biodistribution and elimination of ²²³Ra when using ²²³RaCl₂ as component A and L-arginine as component B of the set for radionuclide therapy

Experimental layout was analogous to Example 1 or 2. In the mouse model, the animals were pre-medicated by oral administration of L-arginine for one week. L-arginine was added to the mouse food once a day. The day before administration of radium, Ca2⁺ was administered orally in the form of a soluble salt. Subsequently, Ra-223 was administered intravenously as a solution of RaCl₂ in physiological NaCl saline. As in previous exemplary embodiments with pre-medication, Ca²⁺ was modulated in the organism and thus Ra uptake and resorption increased in the organism.

### Example 6: Study of biodistribution and elimination of ²²³Ra when using ²²³RaCl₂ as component A and calcitonin as component B of the set for radionuclide therapy

Experimental layout was analogous to Example 3. In the mouse model, the animals were pre-medicated by subcutaneous injection of calcitonin for one week so that its serum concentration is about 5 nM. Two days before administration of Ra-223, Ca²⁺ was administered orally in the form of a soluble salt. Subsequently, Ra-223 was administered intravenously as a solution. As in previous exemplary embodiments, this pre-medication modulated the serum Ca²⁺ concentration and thus Ra-223 uptake increased in the organism.

## Claims

1. A set for radionuclide therapy, comprising a component A and a component B, for use in the treatment of bone diseases by administration of the component B in a dosage ensuring a higher than physiological concentration of a substance affecting Ca²⁺ metabolism in the organism and/or in the compartment treated, and co-administration of the component A, **wherein** the component A comprises a water-soluble radium salt containing at least one radionuclide of radium, and the component B comprises at least one substance affecting Ca²⁺ metabolism in the organism, selected from the group comprising water-soluble salts of alkaline earth metals, calciferols, calcitonins, thyroid and parathyroid gland hormones, amino acids and salts thereof, peptides having a chain length of 2 to 100 amino acids and salts thereof, bisphosphonates of the general formula (I), 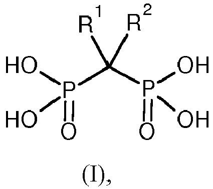 wherein R¹ is -H, -OH, -Cl;
R² is -CH₃, -Cl, -CH₂CH₂NH₂, -(CH₂)₅-NH₂, -(CH₂)₃-NH₂, CH₂CH₂N(CH₃)-(CH₂)₄CH₃; 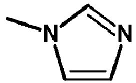 and combinations thereof.

2. The set for use according to claim 1, **characterized in that** the co-administration of the component A and the component B is the administration of the component B first upon reaching the higher than physiological concentration of the substance affecting Ca²⁺ metabolism in the organism, followed by the administration of the component A.

3. The set for use according to claim 1, **characterized in that** the co-administration of the component A and the component B is the administration of the component A first, followed by the administration of the component B upon reaching the higher than physiological concentration of the substance affecting Ca²⁺ metabolism in the organism, wherein the time delay of administration of the component B is within ten biological half-lives of the component A.

4. The set for use according to claim 1, **characterized in that** the co-administration of the component A and the component B is the administration of the component A and of the component B simultaneously.

5. The set for use according to any one of the preceding claims, **characterized in that** Ra radionuclide is selected from the group comprising ²²³Ra, ²²⁴Ra, ²²⁵Ra isotopes.

6. The set for use according to any one of the preceding claims, **characterized in that** the substance affecting calcium metabolism in the organism is selected from water-soluble Mg²⁺, Ca²⁺ Sr²⁺ salts, vitamin D, calcitonin, parathormone, thyroxine, triiodothyronine, zoledronic acid, L-lysine, arginine and salts thereof, peptides having a chain length of 2 to 50 amino acids and salts thereof, or combinations thereof.

7. The set for use according to claim 6, **characterized in that** the component B is selected from MgCl₂, CaCl₂, SrCl₂, vitamin D, calcitonin, parathormone or combinations thereof.

8. The set for use according to any one of the preceding claims, **characterized in that** the component A and/or the component B further comprises up to 99.9 wt.% of a pharmaceutically acceptable excipient, selected from the group comprising preservatives, stabilizers, humectants and/or emulsifiers, solubilizers, salts for osmotic pressure regulation, and buffers.

9. The set for use according to any one of the preceding claims, **characterized in that** the component A and/or the component B further comprises at least one inhibitor of osteoclast activity for the treatment of oncological diseases.

10. The set for use according to any one of the preceding claims, **characterized in that** the bone diseases are selected from bone cancer diseases and bone metastases.

11. The set for use according to any one of the preceding claims, **characterized in that** the administration of the component A is intravenous, and the administration of the component B is oral and/or subcutaneous and/or nasal and/or intravenous.

12. The set for use according to claim 2 in the dosage regimen **characterized by** the administration of the component B at least twice a week at least until a higher than physiological concentration of the substance affecting calcium metabolism in the organism is reached, and by the subsequent single-dose administration of the component A at a higher than physiological concentration of the component B in the organism.

13. The set for use according to claim 12, **characterized in that** after the single-dose administration of the component A, a subsequent administration of the component B takes place, in order to maintain its higher than physiological concentration in the organism during the time interval of untill ten biological half-lives of the component A.
